# EUROPEAN PATENT APPLICATION

(11) **EP 2 196 519 A1**
(43) Date of publication of application: **16.06.2010**
(21) Application number: 08787667.8
(22) Date of filing: 08.07.2008
(51) Int. Cl.: C10L 1/02, C12P 7/00

(54) **METHOD FOR TREATING ORGANIC MATTER AND OBTAINING AND REFINING FUELS FROM SAID MATTER**

(30) Priority: 11.09.2007 ES 200702228
(71) Applicant: Angulo Lafuente, Francisco, La Habana, 39-9º-d 28945 Fuenlabrada (ES)
(72) Inventor: Angulo Lafuente, Francisco, La Habana, 39-9º-d 28945 Fuenlabrada (ES)
(74) Representative: Sanz-Bermell Martinez, Alejandro
(86) International application number: PCT/ES2008/070136
(87) International publication number: WO 2009/034217

(57) **Abstract**

**Method for treating organic materials and obtaining and refining fuels from these materials.**

This comprises a previous elimination of any non-organic materials and grinding the organic materials, as well as possibly adding water until a mass with the suitable thickness is obtained, and also the processes of fermenting the mass at ambient temperature by adding microorganisms for about 5 days, heating up the mass obtained at a temperature of 50°C to 90°C after fermenting for 30-90 min to extract the fats, filtering the resulting liquid mass, extracting the liquid mass obtained from the paraffins and fatty acids obtained, distilling the resulting mass to obtain alcohols and mixing the paraffins with the alcohols obtained in the process in the right proportions.

## Description

### OBJECT OF THE INVENTION

This invention, as stated in the statement of this descriptive report, refers to a method for processing organic materials, which may come from different sources, in which paraffins are obtained by means of a microbiological treatment procedure. After being refined and treated, these paraffins are usable as a fuel, this fuel being suitable for use in internal combustion engines.

The paraffins which will later be refined to be used as fuel are obtained by means of a biological process, normally anaerobic, in which a culture of microorganisms such as bacteria or yeasts is created.

The method being proposed involves different advantages. Unlike fuels obtained from plant crops, such as biodiesel, this invention does not require any large surface areas of crop land, nor water consumption through irrigation, pesticides, harvesting and performing a pre-treatment to be able to be processed, nor are there any limits to the annual production per unit of land or through weather conditions, etc.

Apart from this, the water that is supplied for the process in the invention proposed is partially recoverable and does not need to be high quality water. Hence grey or semi-purified water can be used, without this impairing the biochemical process being carried out.

Since the presence of light is not required in the microbiological treatment being performed, this procedure can be used at different heights inside industrial sheds or underground.

### STATE OF THE ART

There are different processes for treatment of waste from which fuels can be obtained.

WO 2004/060587 describes a method and an apparatus for obtaining solid fuel from organic waste, and includes a biological treatment from which a material with a dampness not over 45% is obtained, which is later on dried for use as a solid fuel.

DE 19838011 describes a method for production of biodiesel from whey obtaining simple oils from the triglycerides found in this whey.

DE 19637909 discloses a procedure for making use of old wood which includes the extraction of lignin, an alcoholic fermentation, and an anaerobic treatment of the substrate obtained, the residual product being used as fertiliser.

ES 2273594 discloses a method for treatment of organic waste which includes a preliminary extraction of inorganic products and a microbiological treatment from which a mixture of alcohols and glycerines is obtained, and later processed, which is optimised according to the method of the invention being proposed.

### DESCRIPTION OF THE INVENTION

According to the method being proposed, the biomass from which the different products are going to be obtained as will be described below, is made up of organic waste, from household rubbish, farms, slaughterhouses, etc.

First of all the inorganic material is separated from the organic material. This inorganic material is discarded, and in general sent or returned to the dump for another kind of treatment.

Then the organic material is ground by mechanical means, for example by means of a blade mill. After grinding the mass to be treated, the degree of fluidity of this mass is measured, as indicated by its water content, and if necessary the amount of water needed is added. The only conditions which this water has to comply with are that it should not contain toxic products and should have a pH close to neutral to be able to facilitate the proliferation of microorganisms. This enables the use of non-drinking water with organic contents inappropriate for human consumption or even for dumping in rivers, on condition that the non-toxicity and pH conditions described above are met. The mass should be homogeneous and pasty.

A culture of microorganisms, normally anaerobically grown, but which can also be aerobic, is added to this mass, to start a fermentation process, decomposing the organic material. For aerobic treatment the addition of blowers is designed to produce bubbling in the reactors, so as to improve the oxygenation of the mass and thus facilitate the development of colonies of micro-organisms. In the case of anaerobic treatment the movement of the mass will be able to be stimulated to ensure more uniform decomposition.

This process is performed to liquefy the fats and be able to extract these. The culture should be at ambient temperature, ideally between 20-40°c. The temperature is only raised to 50-90°c to remove the fats.

The following are some of the microorganisms that can be used, though not implying any exclusivity or limitation thereby:
- Bacteria producing paraffins; these bacteria may be of one or some of the groups of Firmicutes, Enterobacterias, Escherichia coli, Bacteroidetes, Serratia marcescens, amongst others
- Yeasts
- Fungi producing paraffins, such as Mortierella alpina or of the order Mucorales
- Yeast

Although these are not microorganisms, the enzymes aggregated or produced or used by these microorganisms also have an essential function in the decomposition of organic material.

Other milk ferments can also be used, it being possible to use larger-sized multicellular organisms such as worms, which speed up the process of material decomposition.

In a first phase of the process an organic gas is obtained, mainly methane, and when the decomposition is more advanced, the lipids start to be separated, so that these rise to the top of the reactor through being less dense than the decomposing mass.

A certain amount of the paraffins stems from a part of these lipids and another part of the paraffins will be produced by the actual culture of microorganisms; these paraffins are extracted through a separation procedure, for example by decanting.

These paraffins are subjected to a refining process, and will be transformed into fuel by means of a later refining process.

The refining process includes adding oils or light solvents to the paraffinic mass, and/or the alcohols which will be obtained in a later distillation process, after extracting the paraffins. If the temperature of the paraffins is too low, their temperature can be raised to facilitate mixing with the lighter fractions. It has been shown that a mechanical agitation at a temperature of from 70-90 °C for around 15 minutes provides an appropriate mixture.

Alcohols and/or chemical organic solvents can be used as solvents. After completing this process the result is a liquid product mainly consisting of carbon and hydrogen chains which can be used in diesel engine vehicles as an Eco-combustible.

To take advantage of the growth of microorganisms which takes place during the process of decomposition explained above, one part of these is made use of to be added in the previous phase of incorporating said microorganisms.

The product resulting from the extraction is distilled to obtain alcohols.

The process results in different combustible materials such as methane, paraffins and fatty acids, alcohols and solid organic material, which can be used as an agricultural fertiliser or be used as a fuel after being dried.

The fuel is obtained by one of the following methods:
- By mixing the paraffins and fatty acids with the alcohols resulting from the distillation;
- By mixing the paraffins and fatty acids with organic solvents;
- By mixing the paraffins and fatty acids with light oils, such as turpentine essence;
- By means of a transesterification process, through which the molecular links are broken by catalysts;
- Direct use in Diesel engines by preheating to a temperature of about 80°C. This option is particularly recommendable for static engines.

The solid material that has precipitated to the bottom of the reactor is extracted, drained and compacted. The resulting product can be used as an agricultural fertilizer.

The fuel obtained can be given a biocide treatment to prevent the proliferation of colonies of microorganisms degrading the product. This treatment can for example be by heat, radiation, electromagnetic or UV.

## Claims

1. **A method for treating organic materials and obtaining and refining fuels from these materials** comprising a preliminary elimination of non-organic matter and the grinding of organic materials, as well as possibly adding water until a mass with the right thickness is obtained, **characterised by** including the following stages:
• Fermentation of the mass at ambient temperature by adding microorganisms for about 5 days.
• Heating the mass obtained at a temperature of 50°C to 90°C after the fermentation for 30-90 min for the extraction of fats.
• Filtering the resulting liquid mass;
• Extracting the paraffins and fatty acids obtained from the liquid mass produced; and
• Distilling the resulting mass to obtain alcohols;
• Mixing paraffins with the alcohols obtained in the process in the proper proportions.

2. A method, according to claim 1, **characterised in that** this includes previously adding water, this water not being able to contain toxic substances and having to have a pH close to neutral to facilitate the proliferation of microorganisms.

3. A method, according to either of claims 1 and 2, **characterised in that** this includes blowers in the case of aerobic fermentation in order to produce bubbling in the reactors to enable the oxygenation of the mass.

4. A method, according to either of claims 1 and 2, **characterised in that** in the case of anaerobic fermentation the mass is driven in such a way as to produce movement in this in order to ensure uniform decomposition.

5. A method, according to any of the previous claims, **characterised in that** the microorganisms used can comprise one of the following, amongst others:
• Bacteria producing paraffins; these bacteria can be of one or several of the groups *Firmicutes, Enterobacteria, Escherichia coli, Bacteroidetes, or Serratia marcescens,*
• Yeasts
• Fungi producing paraffins, such as *Mortierella alpina* or of the order of *Mucorales.*

6. A method, according to any of claims 1 to 4 or claim 5, **characterised by** using milk ferments.

7. A method, according to any of claims 1 to 6, **characterised by** including a stage prior to microbiological fermentation of treatment by means of multicellular organisms, such as worms.

8. A method according to any of claims 1 to 7, **characterised by** extracting organic gas, mainly methane, from the mass during fermentation, and after this fermentation paraffins and fatty acids and later on alcohols, through distilling the resulting mass.

9. A method, according to claim 8, **characterised in that** the extraction of paraffins and fatty acids is done by decanting.

10. A method, according to any of claims 1 to 9, **characterised in that** part of the microorganisms involved in the fermentation process is separated for its reincorporation to a new treatment cycle.

11. A method, according to any of claims 1 to 9, **characterised in that** a liquid fuel suitable for internal combustion engines is obtained by mixing paraffins and fatty acids with the alcohols produced by the distillation.

12. A method, according to any of claims 1 to 9, **characterised in that** a liquid fuel suitable for internal combustion engines is obtained by mixing the paraffins and fatty acids obtained with organic solvents.

13. A method, according to any of claims 1 to 9, **characterised in that** a liquid fuel appropriate for internal combustion engines is obtained by mixing paraffins and fatty acids with light oils, such as turpentine oil.

14. A method, according to any of claims 1 to 9, **characterised in that** a liquid fuel suitable for internal combustion engines is obtained by a transesterification process.

15. A method, according to any of claims 1 to 9, **characterised by** the use of paraffins and fatty acids as a liquid fuel suitable for internal combustion engines by heating up to a temperature of about 80 °C.

16. A method, according to any of claims 11 to 13, **characterised by** including mechanical agitation of the paraffins and fatty acids with the products to be mixed at a temperature of 70-90 °C for a period of 15 min.

17. A method, according to any of claims 1 to 16, **characterised by** draining the solid material stemming from the process which can be used as a farming fertiliser or solid fuel.

18. A method, according to any of claims 1 to 16, **characterised by** putting the paraffins or the fuel obtained through a biocide treatment.
